# EUROPÄISCHE PATENTANMELDUNG

(11) **EP 0 893 093 A1**
(43) Veröffentlichungstag der Anmeldung: **27.01.1999**
(21) Anmeldenummer: 97810533.6
(22) Anmeldetag: 25.07.1997
(51) Int. Cl.: A61B 5/042, A61N 1/05

(54) **Katheter, zum endokardialen Abtasten von Herzpotentialen**

(71) Anmelder: Sulzer Osypka GmbH, 79639 Grenzach-Wyhlen (DE)
(72) Erfinder: Baumgartner, Daniel, D-79639 Grenzach-Wyhlen (DE)
(74) Vertreter: Heubeck, Bernhard

(57) **Zusammenfassung**

Der Katheter zum endokardialen Abtasten von Herzpotentialen - nämlich ein Mutterkatheter (1) - umfasst an seinem distalen Ende mindestens zwei Elektrodenpole (11a, 11b, 11c). Mindestens ein im Katheter vom proximalen zum distalen Ende sich erstreckendes Lumen (121, 122) enthält einen Seitenkatheter (2), der in diesem verschiebbar angeordnet ist. Für jeden Seitenkatheter ist eine Austrittsöffnung (121', 122') seitlich oder am distalen Ende des Mutterkatheters vorgesehen.

## Beschreibung

Die Erfindung betrifft einen Katheter zum endokardialen Abtasten von Herzpotentialen gemäss Oberbegriff von Anspruch 1.

Bei diagnostischen Eingriffen am Herzen werden zur Ableitung von elektrophysiologischen Signalen mehrere Katheter verwendet: Ein erster Katheter für die Ableitung am HIS-Bündel, ein zweiter für die Ableitung im hohen Atrium und ein dritter für die Ableitung im rechten Ventrikel. Bei einer Diagnose dieser Art muss die Beinvene an drei Stellen punktiert werden, und es sind entsprechend drei Einführschleusen erforderlich. Es ist dabei in der Regel ein Klinikaufenthalt des Patienten nötig, der länger als 24 Stunden dauert.

Aufgabe der Erfindung ist es, einen Herzkatheter zur elektrophysiologischen Diagnose zu schaffen, mit dem ein Eingriff durchführbar ist, der den Patienten weniger belastet. Diese Aufgabe wird mit dem in Anspruch 1 definierten Katheter gelöst.

Der Katheter zum endokardialen Abtasten von Herzpotentialen - nämlich ein Mutterkatheter - umfasst an seinem distalen Ende mindestens zwei Elektrodenpole. Mindestens ein im Katheter vom proximalen zum distalen Ende sich erstreckendes Lumen enthält einen Seitenkatheter, der in diesem verschiebbar angeordnet ist. Für jeden Seitenkatheter ist eine Austrittsöffnung seitlich oder am distalen Ende des Mutterkatheters vorgesehen.

Die abhängigen Ansprüche 2 bis 10 beziehen sich auf vorteilhafte Ausführungsformen des erfindungsgemässen Katheters.

Nachfolgend wird die Erfindung anhand der Zeichnungen erläutert. Es zeigen:
- Fig. 1: distaler Bereich eines erfindungsgemässen Mutterkatheters,
- Fig. 2: eine Austrittstelle im Mutterkatheter für einen Seitenkatheter,
- Fig. 3: einen Seitenkatheter,
- Fig. 4: Detail mit Querschnitten durch den Mutterkatheter sowie die Seitenkatheter,
- Fig. 5: die Hälfte eines geöffneten Griffs zum Mutterkatheter,
- Fig. 6: den geschlossenen Griff der Fig.5 und
- Fig. 7: eine zweite Ausführungsform des Griffs.

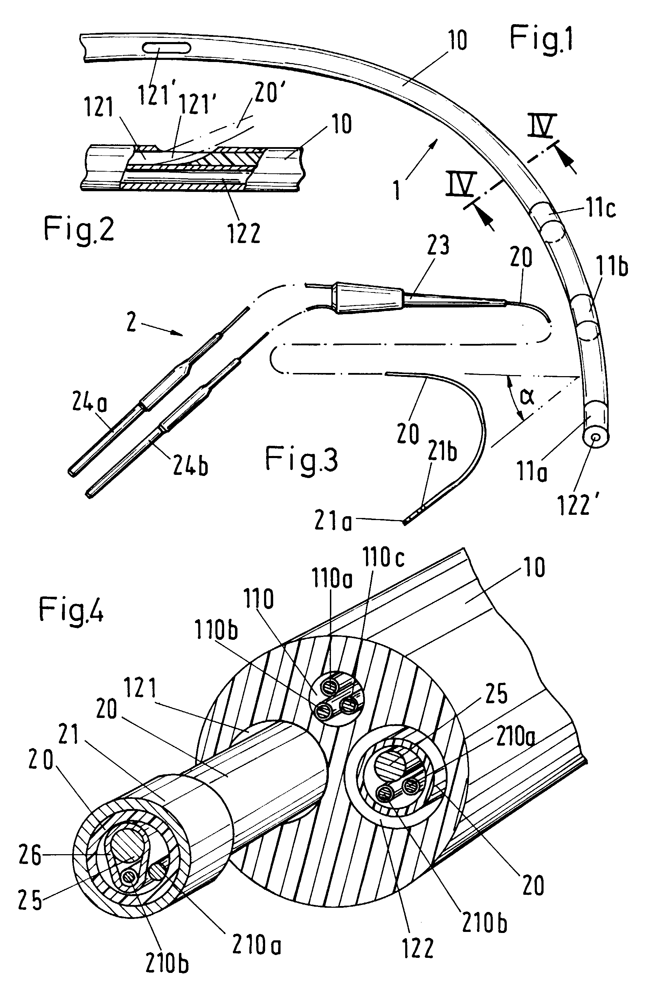

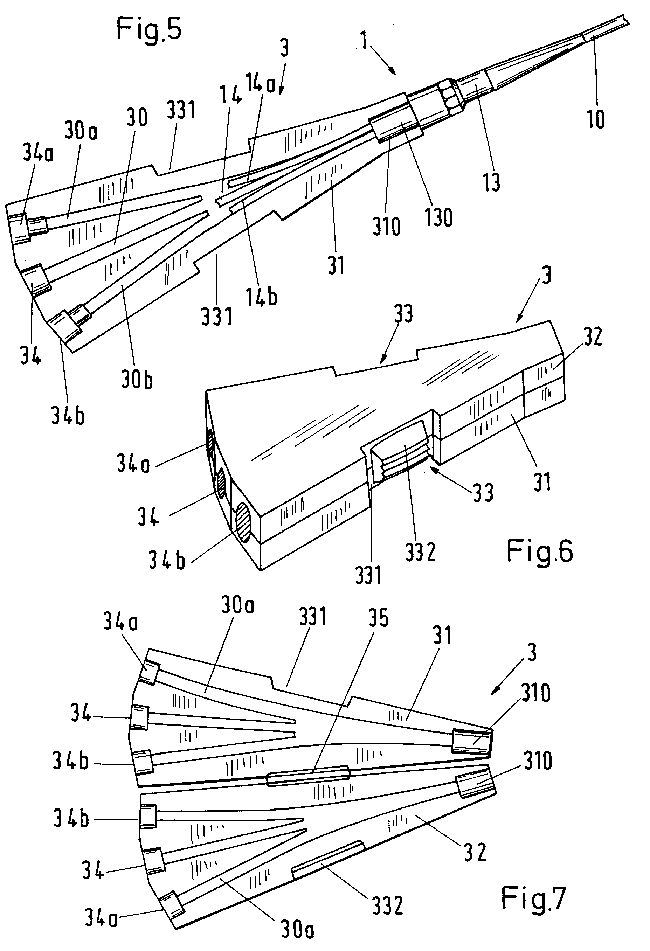

Der Mutterkatheter 1 in Fig.1 umfasst einen mehrlumigen Schlauch 10 und Ringelektroden 11a, 11b und 11c an dem distalen Ende. Die an der distalen Spitze angeordnete Elektrode 11a enthält eine Austrittstelle 122' für einen Seitenkatheter 2 (siehe Fig.3). Zwei Lumen 121, 122 (siehe Fig.2) für je einen Seitenkatheter 2 erstrecken sich im Mutterkatheter 1 von einem Übergangsstück 13 am proximalen Ende (siehe Fig.5) zu dem distalen Ende. Für den weiteren Seitenkatheter 2 ist eine Austrittstelle 121' seitlich am Mutterkatheter 1 eingelassen.

Fig.2 zeigt die beiden Lumen 121 und 122 in einem ausschnittsweise vorgenommenen Längsschnitt durch den Schlauch 10. Strichpunktiert ist die Lage 20' eines ausgeschobenen Seitenkatheters 2 dargestellt. Fig.3 zeigt den Seitenkatheter 20, der einen Schlauch 20, Elektrodenpole 21a und 21b, entsprechende Anschlussstecker 24a und 24b sowie einen Griff 23 am proximalen Ende des Schlauchs 20 umfasst.

Der Mutterkatheter 1 enthält - siehe Fig.4 - ein weiteres Lumen 110, in dem Zuleitungsdrähte 110a, 110b, 110c für die Elektrodenpole 11a, 11b, 11c angeordnet sind.

Jeder der Seitenkatheter 2 umfasst folgende Komponenten: mindestens zwei hülsenförmige Elektrodenpole 21a, 21b, einen Kunststoffschlauch 20 und Drähte in dem Kunststoffschlauch, nämlich isolierte Zuleitungsdrähte 210a, 210b zu den Elektrodenpolen sowie ein Stützdraht 25 aus federelastischem Werkstoff. Der Kunststoffschlauch 20 besteht beispielsweise aus PTFE oder Polyimid mit PTFE-Beschichtung, der federelastische Werkstoff vorzugsweise aus Nitinol.

Die Elektrodenpole 21a, 21b sind auf den Schlauch 20 aufgeschrumpft. Damit beim Aufschrumpfen eines Pols 21 durch die mechanische Einwirkung kein elektrischer Kontakt zwischen den Zuleitungsdrähten 210a, 210b entstehen kann, ist im Bereich des Pols 21 ein Schlauchstück 26 vorgesehen, wie es in Fig.4 dargestellt ist. Dieses Schlauchstück 26 bildet eine Trennwand zwischen den beiden Zuleitungsdrähten 210a und 210b.

Der Mutterkatheter 1 weist einen vorgeformten distalen Bereich auf, der in der Längsrichtung des Katheters geeignet gebogen ist, so dass seine Form den Wandkonturen des zu behandelnden Herzteils entspricht.

In einer nicht dargestellten Ausführungsform ist ein Zugdraht in ein weiteres Lumen eingelegt; mit diesem lässt sich die Form des Mutterkatheters 1 steuerbar verändern.

Auch die Seitenkatheter 2 sind mit Vorteil vorgeformt. Der distale Bereich des Seitenkatheters kann, wie in Fig.3 gezeigt, eine permanente Vorformung mit einem Winkel α von beispielsweise 45° aufweisen.

Die Figuren 5 bis 7 betreffen einen Griff 3, der am proximalen Ende des Mutterkatheters 1 angeordnet ist. Dieser Griff 3 weist Eintrittsöffnungen 34a, 34b für die in den Mutterkatheter 1 einschiebbaren Seitenkatheter 2 auf. Mit Vorteil ist der Griff 3 lösbar am Mutterkatheter 1 befestigt und zwar öffenbar, so dass im Griff liegende Kanäle 30, 30a, 30b für Reinigungszwecke freigelegt werden können. Während die Katheter für einen einmaligen Gebrauch vorgesehen sind, kann ein lös- und öffenbarer Griff 3 wiederverwendet werden.

Fig.5 zeigt den einen Teil 31 eines zweiteiligen Griffs 3. An dessen vorderen Ende ist das proximale Übergangsstück 13 des Mutterkatheters 1 mit einem zylindrischen Teil 130 in eine entsprechende Nut 310 des Griffs 3 eingelegt. In Fortsetzung der Lumen 110, 121 und 122 (Fig.4) gehen vom Übergangsstück 13 Schläuche 14, 14a und 14b aus (nur teilweise dargestellt), die bis zu den Eintrittsöffnungen 34, 34a bzw. 34b führen. In die Schläuche 14a und 14b lassen sich die Seitenkatheter 2 einschieben. In dem Schlauch 14 sind die Zuleitungsdrähte 110a, 110b, 110c des Mutterkatheters 1 enthalten. Die Schläuche sind in den Kanälen 30, 30a und 30b eingelegt.

Auf den Griffteil 31 wird ein entsprechender Teil 32 - siehe Fig.6 - aufgesetzt. Mittels Riegeln 332, die mit dem Teil 32 fest verbunden sind und die in Nuten 331 des Teils 31 einschiebbar sind, werden die beiden Teile 31 und 32 lösbar miteinander verbunden. Die Riegel 332 bestehen mit Vorteil aus einem elastischen Werkstoff, der die Ausbildung einer Schnappverbindung ermöglicht.

Fig.7 zeigt eine zweite Ausführungsform eines wiederverwendbaren Griffs 3: zwei Teile 31 und 32, die durch ein Scharnier 35 miteinander verbunden sind. Wie bei der ersten Ausführungsform gemäss den Figuren 5 und 6 ist ein aus einem Riegel 332 und einer Nut 331 bestehender Schnappverschluss vorgesehen.

Die nutzbare Länge des Mutterkatheters 1 beträgt vorzugsweise rund 1 m; die nutzbaren Längen der Seitenkatheter 2 sollen rund 25 cm grösser sein.

Die proximalen Katheterenden sind mit hämostatischen Ventilen versehen. Über Sideport-Anschlüssen an den hämostatischen Ventilen lassen sich die Lumen 121, 122 der Seitenkatheter 2 spülen.

Der erfindungsgemässe Katheter 1 eignet sich für die Durchführung von elektrophysiologischen Herzdiagnosen. Dabei dient der Mutterkatheter 1 zur Signalableitung am HIS-Bündel, ein erster Seitenkatheter 2 zur Signalableitung im hohen Atrium und ein zweiter Seitenkatheter 2 zur Signalableitung im rechten Ventrikel.

Ist die Form des Mutterkatheters 1 mittels eines Zugdrahts steuerbar veränderbar, so kann der diagnostische Eingriff über die obere Hohlvene durchgeführt werden.

## Patentansprüche

1. Katheter zum endokardialen Abtasten von Herzpotentialen mit mindestens zwei Elektrodenpolen (11a, 11b, 11c) an dessen distalen Ende, dadurch gekennzeichnet, dass mindestens ein vom proximalen zum distalen Ende sich erstreckendes Lumen (121, 122) im Katheter - dem sogenannten Mutterkatheter (1) - enthalten ist, dass in diesem Lumen ein zweiter oder ein weiterer, Elektrodenpole (21a, 21b) aufweisender Katheter - ein sogenannter Seitenkatheter (2) - verschiebbar angeordnet ist und dass für jeden Seitenkatheter eine Austrittsöffnung (121', 122') seitlich oder am distalen Ende des Mutterkatheters vorgesehen ist.

2. Katheter nach Anspruch 1, dadurch gekennzeichnet, dass ein weiteres Lumen (110) im Mutterkatheter (1) enthalten ist, in dem Zuleitungsdrähte (110a, 110b, 110c) für die Elektrodenpole (11a, 11b, 11c) des Mutterkatheters angeordnet sind.

3. Katheter nach Anspruch 1 oder 2, dadurch gekennzeichnet, dass jeder der Seitenkatheter (2) folgende Komponenten umfasst: mindestens zwei hülsenförmige Elektrodenpole (21a, 21b), einen Kunststoffschlauch (20), insbesondere aus PTFE oder Poliimid mit einer PTFE-Beschichtung, auf dem die Elektrodenpole aufgebracht sind, und Drähte in dem Kunststoffschlauch, nämlich Zuleitungsdrähte (210a, 210b) zu den Elektrodenpolen sowie einen Stützdraht (25) aus federelastischen Werkstoff, vorzugsweise aus Nitinol.

4. Katheter nach einem der Ansprüche 1 bis 3, dadurch gekennzeichnet, dass die Anzahl der Seitenkatheter (2) zwei beträgt und dass eine seitliche sowie eine am distalen Ende angeordnete Austrittsöffnung (121', 122') für die Seitenkatheter vorgesehen sind.

5. Katheter nach einem der Ansprüche 1 bis 4, dadurch gekennzeichnet, dass am proximalen Ende des Mutterkatheters (1) ein Griff (3) angeordnet ist und dass der Griff Eintrittsöffnungen (34a, 34b) für die Seitenkatheter (2) aufweist.

6. Katheter nach Anspruch 5, dadurch gekennzeichnet, dass der Griff (3) lösbar am Mutterkatheter (1) befestigt ist und dass er öffenbar ist, so dass im Griff liegende Kanäle (30a, 30b), die für die Seitenkatheter (2) vorgesehen sind, freilegbar sind.

7. Katheter nach einem der Ansprüche 1 bis 6, dadurch gekennzeichnet, dass der Mutterkatheter (1) einen vorgeformten distalen Bereich aufweist, der in der Längsrichtung des Katheters geeignet gebogen ist, so dass seine Form den Wandkonturen des zu behandelnden Herzteils entspricht, und dass ferner auch die Seitenkatheter (2) Vorformungen aufweisen können.

8. Katheter nach Anspruch 7, dadurch gekennzeichnet, dass die Form des Mutterkatheters (1) mittels eines Zugdrahts steuerbar veränderbar ist.

9. Katheter nach einem der Ansprüche 1 bis 8, dadurch gekennzeichnet, dass die nutzbare Länge des Mutterkatheters (1) rund 1 m beträgt und dass die nutzbaren Längen der Seitenkatheter (2) rund 25 cm grösser sind.

10. Katheter nach einem der Ansprüche 1 bis 9, dadurch gekennzeichnet, dass die Lumen (121, 122) der Seitenkatheter (2) über Sideport-Anschlüsse spülbar sind.

11. Verfahren zur elektrophysiologischen Herzdiagnose mit einem Katheter gemäss einem der Ansprüche 1 bis 10, dadurch gekennzeichnet, dass der Mutterkatheter für eine Signalableitung am HIS-Bündel, ein erster Seitenkatheter für eine Ableitung im hohen Atrium und ein zweiter Seitenkatheter für eine Ableitung im rechten Ventrikel verwendet werden.

12. Verfahren nach Anspruch 11, dadurch gekennzeichnet, dass die Form des Mutterkatheters mittels eines Zugdrahts steuerbar veränderbar ist und dass der diagnostische Eingriff über die obere Hohlvene durchgeführt wird.
